(19) **Europäisches Patentamt**

**European Patent Office**

**Office européen des brevets**

(11) **EP 0 430 991 B1**

(12) **EUROPEAN PATENT SPECIFICATION**

(45) Date of publication and mention
of the grant of the patent:
**23.10.1996 Bulletin 1996/43**

(21) Application number: **89909276.1**

(22) Date of filing: **25.07.1989**

(51) Int Cl.$^6$: **C12Q 1/32**, C12N 9/99

(86) International application number:
**PCT/US89/03217**

(87) International publication number:
**WO 90/01067 (08.02.1990 Gazette 1990/04)**

(54) **REAGENT FOR USE IN LD-1 ASSAY**

REAGENZ ZUR VERWENDUNG IM LD-1-TESTVERFAHREN

REACTIF UTILISE POUR LE TITRAGE DE LD-1

(84) Designated Contracting States:
**AT BE CH DE FR GB IT LI LU NL SE**

(30) Priority: **25.07.1988 US 224424**

(43) Date of publication of application:
**12.06.1991 Bulletin 1991/24**

(73) Proprietors:
- **BOEHRINGER MANNHEIM CORPORATION**
  **Indianapolis Indiana 46250 (US)**
- **WAKE FOREST UNIVERSITY**
  **Winston-Salem, NC 27103 (US)**

(72) Inventors:
- **SHIHABI, Zak**
  **Winston-Salem, NC 27103 (US)**
- **WU, Yih-Shiong**
  **Carmel, IN 46032 (US)**
- **SIMMONS, Mark**
  **Carmel, IN 46032 (US)**

(74) Representative: **Jung, Michael, Dr. et al**
**Boehringer Mannheim GmbH**
**Patentabteilung**
**Sandhofer Strasse 116**
**68305 Mannheim (DE)**

(56) References cited:
**JP-A-62 278 997**

- **PATENT ABSTRACTS OF JAPAN, vol. 14, no. 180 (C-708)[4123], 11th April 1990; & JP-A-2 27 997 (KOKUSAI SHIYAKU K.K.) 30-01-1990**
- **CLINICAL CHEMISTRY, vol. 36, no. 10, 1990, pages 1819-1822, Winston-Salem, NC, US; T.A. ONIGBINDE et al: "Clinical evaluation of an automated chemical inhibition assay for lactate dehydrogenase isoenzyme 1"**
- **CLINICAL CHEMISTRY, vol. 35, no. 6, 1989, page 1119, abstract no. 251, Winston-Salem, NC, US; Y.-S. WU et al.;**
- **CLINICAL CHEMISTRY, vol. 36, no. 6, 1990, page 1135, abstract no. 0853, Winston-Salem, NC, US; T.A. ONIGBINDE et al.: "Enzyme kinetics of lactate dehydrogenase isoenzymes and the mechanism of chemical inhibition for the BMD LD-1 assay"**
- **Clinical Chemistry, Vol. 31, No. 7, 1985, TANISHIMA et al, "Activity of Lactate Dehydrogenase Isoenzymes LD-1 and LD-2 in Serum as Determined by using an Inhibitor of the M-Subunit", see pages 1175-1177**
- **Clinical Chemistry, Vol. 33, No. 6, 1987, ABBOTT et al, "A New Method for the Selective Determination of Lactate Dehydrogenase Isoenzyme I (LD-1) using Sodium Perchlorate". see pages 991-992, Abstract 544.**

## Description

Field of the Invention

This invention relates generally to the measurement of the components of blood serum and more particularly to the measurement of serum lactate dehydrogenase.

Background of the Invention

Lactate dehydrogenase (LDH) is an enzyme present in the human body in the form of five different isoenzymes which have been designated LD-1, LD-2, LD-3, LD-4 and LD-5 based on their relative electrophoretic migration toward an anode, with LD-1 being the most anodic.

These enzymes are tetrameric proteins composed of two subunits: type "M", prevalent in skeletal muscle, and type "H", prevalent in the heart. The respective subunit combinations of LD-1 through LD-5 are H(4), H(3)M, H(2)M(2), HM(3) and M(4).

These five isoenzymes catalyze the same chemical reaction, but are distributed in different regions of the body. Damage to cells in any particular body region, as may occur from a myocardial infarction for example, releases the particular LDH isoenzymes present in that region into the bloodstream.

By withdrawing a sample from the bloodstream, the types of LDH present in the blood can be determined and the region of the body which has been damaged identified accordingly. Because all five of the LDH isoenzymes are ordinarily present in the blood in varying amounts at all times, an unusual elevation in the concentration of one of the LDH isoenzymes will indicate that a particular region of the body has been damaged.

Because the heart contains LD-1 and LD-2, the occurrence of a myocardial infarction can be determined by performing a diagnostic test to see whether or not the amount of either of these isoenzymes in the bloodstream is elevated. LD-1 is a particularly preferred marker for myocardial infarction as the elevation of LD-1 in blood, measured within 48 hours after the onset of chest pains, confirms the occurrence of a heart attack with high accuracy. Recently, studies have suggested that changes in the ratio of LD-1 to total LDH activity provide a reliable indication of the severity and progress of a recent myocardial infarction.

A preferred method for measuring the amount of LD-1 in blood serum is the colorimetric method in which LD-1 catalyzes the conversion of two substrates, lactate and nicotinamide adenine dinucleotide (NAD) to pyruvate and the reduced form of NAD (NADH). These chemical changes result in a color change in a solution which can be measured with a colorimeter. When it is desired to obtain a direct measure of LD-1 activity in blood serum colorimetrically for determination of LD-1 concentration or for determination of its ratio to total LDH concentration or activity, LD-2 through LD-5 must first be inactivated so that only LD-1 will catalyze the lactate and NAD co-substrates.

An automated essay for the determination of lactate dehydrogenase LD-1 is suggested in Eckfeldt, J.H., Kershaw, M.J., and Lewis, L.A., Clin. Chem. 30, 1821 (1984). In this method, blood serum must preferably be pre-incubated for 20 minutes in a solution of lactate and cyclohexylaminopropane sulfonic acid (CAPS). After pre-incubation, this solution is mixed with a second solution containing NAD, and, after a delay of 2 minutes, the resulting reaction is measured colorimetrically.

Takizawa et. al., Clin. Chem. 29 (1983) which was considered and referenced by Eckfeldt, et al, supra, suggests inactivating individual LDH isoenzymes with shorter or no pre-incubation periods. Takizawa, however, deals with individual LDH isoenzymes and does not teach how to measure blood serum LD-1 in a rapid colorimetric assay by inactivating LD-2 through LD-5 simultaneously.

Japanese patent application 86 122,193 by latron Laboratories (Japanese patent publication JP 62 278,997, published December 3, 1987) describes reagents for measuring LDH isoenzymes. The reagent is a combination of alkali buffers, such as CAPS and protein denaturing agents, such as urea, thiourea, guanadine, and thiocyanate salts of trichloroacetic acid. There is no showing or suggestion in the latron publication of the use of a protein denaturing agent and a thiocyanate anion in combination to inhibit the activity of LD-2 through LD-5.

The inactivating effects on LD-2 through LD-5 are known from, for example, Gubernieva, L.M., Safronova, E.E. and Malakhov, V.N. Biochemistry, 39/6(II) (1020-1033), 1975; McQueen, M.J., Ann. Clin. Biochem, 11/3 (75-80) 1974; and Foo, A.Y., Nemesanszky, E., and Rosalki, S.B. Ann. Clin. Biochem. 18 (4) 232-235, 1981. There is no showing or suggestion in any of these prior disclosures of the use of a protein denaturing agent and a thiocyanate anion in combination to inhibit the activity of LD-2 through LD-5.

US Patent No. 4,224,406, issued September 23, 1980, to Gomez and Wicks suggests the use of antibodies to remove LD-2 through LD-5. Japanese patent publication 53,046,090, published April, 25, 1978, suggests the use of a reduced type of NAD to inhibit LDH activity.

T.W. Thannhauser et al., Analytical Biochemistry 138, 181-188 (1984) discloses the use of guanidine thiocyanate in high concentrations to denature a protein in order to make disulfide bonds accessible for quantitative cleaving by

Na$_2$SO$_3$. An ingredient for protecting LD-1 from inhibition is not mentioned.

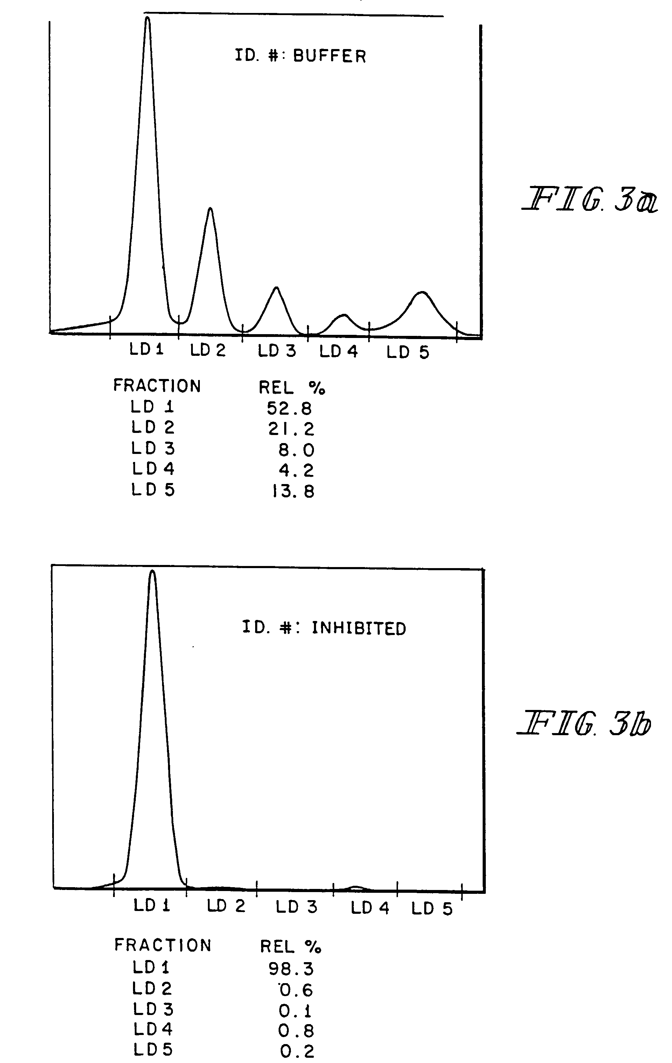

JP-A-62278997 teaches an optimum pH of 10.15 for the inactivation of LD-2, LD-3, LD-4, LD-5 in the presence of a protein denaturant. When lowering the pH inactivation is described to become worse and the reagent is then unusable. Table 1 and figure 3 show that at pH values lower then 10.15 at least LD-2 becomes more and more active.

In the modern medical industry many tests are performed with the use of automated analyzers which are characterized by the use of small amount of samples and reagents and relatively short reaction times. There is a need for an improved reagent for the inhibition of the activity of LD-2 through LD-5 which functions with sufficient speed and simplicity to be compatible with automated clinical analyzers. There is also a need for such a reagent which can operate at a neutral to alkaline pH in order to obtain increased shelf-life of the reagent in commerce.

SUMMARY OF THE INVENTION

It is an object of the present invention to provide an effective means for inhibiting the activity of LD-2 through LD-5 in a neutral to alkaline environment.

It is another object of the present invention to provide a reagent for the inhibition of LD-2 through LD-5 which operates with sufficient speed and simplicity as to be useful in automatic clinical analyzers.

These and other objects are achieved by the present invention which, in one aspect, relates to a reagent for inactivating LD-2 through LD-5 in neutral to alkaline environments which comprises: (1) the reaction product of (a) guanidine and (b) a thiocyanate anion, the reaction product being present in a concentration of about 240 mM and (2) lactate; and (3) a buffer to stabilize the pH at 9.5 to 9.6.

In another aspect the invention relates to a kit for the determination of LD-1 activity in a specimen which contains LD-1 through LD-5, the kit comprising the above reagent and also a second reagent capable of reacting with LD-1 in a measurable manner.

In yet another aspect the invention relates to a method for determining LD-1 activity in a specimen containing LD-1 through LD-5, the method comprising applying the above reagent to at least a portion of the specimen to inhibit the activity of LD-2 through LD-5 and applying a second reagent to at least the portion of the specimen to react in a measurable manner with the LD-1 which remains active and determining the LD-1 activity in the specimen as a function of the measurable reaction of the second reagent.

The invention furthermore concerns a method for determining the LD-1 activity of a specimen, which method comprises:

separating the specimen into first and second portions;
applying the reagent of claim 1 to the first portion to inhibit the activity of LD-2, LD-3, LD-4 and LD-5;
applying a second reagent to the first and second portions to react in a measurable manner to the LD activity in both the first and second portions; and
determining the LD-1 activity in the specimen as a ratio of total LD activity in the specimen as a function of the measurable reaction of the second reagent with the first and second portions.

Also the invention relates to a kit for determining LD-1 activity as a ratio of total LD activity in a specimen, the kit comprising the reagent of claim 1 for inactivating LD-2, LD-3, LD-4 and LD-5 in one of two portions of the specimen and a second reagent capable of reacting in a measurable manner with LD-1, LD-2, LD-3, LD-4 and LD-5 in both portions of the specimen whereby the LD-1 activity as a ratio of total LD activity in the specimen can be determined as a function of the measurable reaction of the second reagent with both portions of the specimen.

BRIEF DESCRIPTION OF THE DRAWINGS

FIG. 1 is a linear regression graph which shows the correlation between the observed LD-1 activity and the expected LD-1 activity when the reagent of the present invention is evaluated by testing for known amounts of activity.

FIG. 2 shows the results of an electrophoretic analysis which confirms the specificity of the reagent of the present invention.

FIG. 3a illustrates the LDH profiles of blood samples from patients with abnormal LDH profiles.

FIG. 3b shows the LDH profiles of the same samples as shown in FIG. 3a after the reagent of the present invention has been added, indicating the specificity of the reagent of the present invention.

FIG. 4 is a linear regression graph which compares the accuracy of the reagent of the present invention with an isomune method of measurement.

DETAILED DESCRIPTION

The present invention is based on the discovery that LD-2 through LD-5 can be quickly and effectively inactivated in a specimen containing LD-1 through LD-5 by a reagent according to claim 1. It has been shown that the protein denaturing agent by itself will not inactivate LD-2 through LD-5 at the desired pH. It has also been shown that other salts of thiocyanate will not alone provide the desired results in that pH range. However, when the protein denaturing agent and the thiocyanate anion are combined, the reaction product forms a selective and effective reagent for inactivating LD-2 through LD-5.

The reagent, which operates to inactivate any LDH moiety containing an "M" aspect can contain any useful protein denaturing agent which works by breaking hydrogen bonds. Examples of suitable such agents are urea, guanidine, thiourea and mixtures thereof. Guanadine has proven to be the preferred such agent because of its commercial convenience.

The thiocyanate anion is normally provided by a thiocyanate salt such as sodium thiocyanate, potassium thiocyanate or mixtures of the two, although any source of thiocyanate anion is suitable for use in the invention.

The concentration of guanadine thiocyanate is about 240 mM at a pH of 9.5.

Lactate has been found to protect the LD-1 isoenzyme from reduction in activity by the reagent. The reagent without lactate has been found to completely inhibit the activity of LD-2 through LD-5 and to suppress slightly the activity of LD-1. However, the addition of Lactate to the reagent will avoid this suppressing effect.

A concentration of about 90 mM of lactate is preferred for use in combination with the amount of guanadine thiocyanate for operation at pH 9.5.

A buffer may also be used with the reagent. A buffer which has been found to be useful is a combination of 2-amino-2-methyl-1,3-propanediol (AMP) and trihydroxymethylaminomethane hydrochloride (Tris), although any suitable buffer may be used.

If the AMP/Tris buffer is used, the AMP portion may be present in amounts up to about 300 mM and the Tris portion may be present in amounts up to about 200 mM. When the reagent operates at pH 9.5, the buffer comprises about 200 mM AMP and about 77 mM Tris.

In operation the reagent is applied to a specimen containing LDH isoenzymes and stirred. Subsequently a second reagent comprising a combination of lactate and NAD is added and any color change of the second reagent is measured. In one application of this invention the color change caused to the second reagent by the untreated specimen is compared with the color change caused to the second reagent by the treated specimen so that an estimate can be made of the ratio of LD-1 to the total LDH content. The ratio is normally calculated from the results of a spectrophotometer reading. These operations may be done manually or with the help of machines as is described in connection with the examples, below.

The reagent may be supplied commercially as a single component, but may also be supplied as a part of a kit which also includes the second reagent (lactate and NAD) and, perhaps, equipment such as pipetts, vials, etc.

EXAMPLES

The present invention will now be described in greater detail in connection with the following examples which are intended to be illustrative and not limiting.

Example 1

Materials and Methods

A. Materials:

1. Reagent I. contains Lithium L-Lactate (90mM), guanadine thiocyanate (240 mM), Tris buffer (112.6 mM) at pH 9.5.
2. Reagent II. contains $Na_2SO_4$ (40 mM) and NAD (53.76 mM).
Reaction mixture is a combination of 2.0 ml of R-1, 0.5 ml of R-2 which has a final reaction pH of 8.9
3. LD isoenzymes. Pure LD-1, LD-2, LD-3 and LD-5 individual isoenzymes were obtained from Aalto of Escondido, CA. Patient serum samples were obtained from a local hospital.

B. Procedures Using Automated Analyzer
Tests were performed on a Boehringer Mannheim/Hitachi 705 analyzer. The analyzer was given the following parameters:

Assay Code: Rate - 20-26 (equivalent to 84.62 seconds to 198.70 seconds)
Sample Volume: 12 µl
R1 Volume: 400 µl
R2 Volume: 100 µl
Wavelength 1: 376 nm
Wavelength 2: 340 nm
Temperature: 37 degrees C.
Factor: -10470

C. Manual Procedure

Instrument: Spectrophotometer
Temperature: 37 degrees C.
Wavelength: 340 nm

1. Zero spectrophotometer with distilled or deionized water (DI HOH)

2. Pipette the following reagents in a cuvette:

|  | Sample | Blank |
|---|---|---|
| R1 | 2.0 ml | 2.0 ml |
| Sample | 0.060 ml | -- |
| DI HOH | --- | 0.060 |

3. Mix and equilibrate sample to 37 degrees C.

4. Pipette and add 0.5 ml of R2 into sample test tube. Mix and measure optical density change at 340 nm for about 6 minutes. Calculate A/min. from 2 to 4 minutes. This is A/min for the sample.

5. Repeat steps 2 to 4 for the blank using 0.060 ml DI HOH as sample. This is A/min. for the blank.

6. Calculate for U/L (U/L = units of LDH activity per liter)

$$U/L = \frac{(A/min\ Sample - A/min\ blank) \times 1000 \times 2.56}{6.22 \times light\ path \times 0.06}$$

D. Protocol for the Electrophoretic Analysis

1. About 630 to 1,000 µl of individual LD-1, LD-2, LD-3, LD-4, LD-5 and abnormal patient serum samples were used in the electrophoretic analysis.

2. 12 µl sample was added to 400 ml R1 and incubated for 4 minutes at 37 degrees C to inhibit LD-2 to LD-5 to achieve an inhibited sample.

3. Another 12 µl sample was added to 400 ul of Tris buffer (112.6 mM, pH at 8.9-9.0) and incubated for 4 minutes at 37 degrees C to achieve a control sample.

4. Both the inhibited and control samples were applied to the Beckman Paragon Gel and the electrophoresis was run according to the Beckman procedure specified for the gel using a Beckman Paragon Electrophoresis instrument from Beckman Instrument.

E. Method for Comparison
Sixty-eight patient samples with LD-1 activity from 30 to 800 U/L were tested to compare the selectivity of R1 with the immunochemical separation method Roche's Isomune LD-1 reagent was employed for the immunochemical separation.

F. Experimental Results

1. LD isoenzymes were tested using the Automated Analyzer procedure described above. The purpose of the test is to determine whether reagent of the present invention selectively inactivates LD-2 through LD-5 while leaving LD-1 active. The results shown in Table I, below indicate that the reagent of the present invention is selective in this manner. LD-1 isoenzyme was tested at several concentrations. An increase in LD-1 activity was noted in some test results.

TABLE I

| Samples | Total LDH Reagent | Activity After R1 Treatment |
|---------|-------------------|------------------------------|
| LD-1 | U/L | U/L |
| 1x | 178 | 176 (98%) |
| 2x | 352 | 352 (100%) |
| 4x | 695 | 698 (100.4%) |
| 6x | 1016 | 1037 (102%) |
| LD-2 | 714 | 8 (1.1%) |
| LD-3 | 1011 | 1 ( 0%) |
| LD-4 | 1026 | 1 ( 0%) |
| LD-5 | 1699 | 4 ( 0%) |

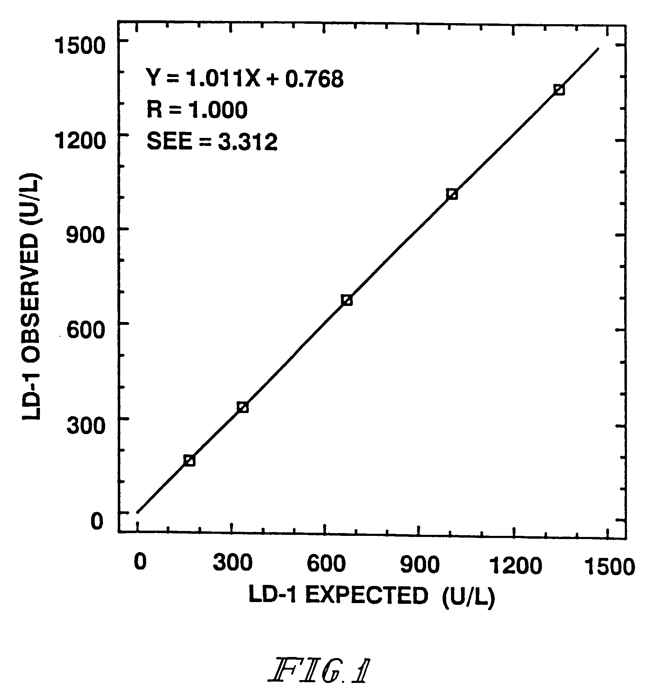

FIG. 1 shows that there is linearity up to at least 1,000 U/L between the observed activity of LD-1 and the expected activity of LD-1. FIG. 1 shows a correlation coefficient ("R") of 1.000 and a standard error of estimate of 3.312 ("SEE")

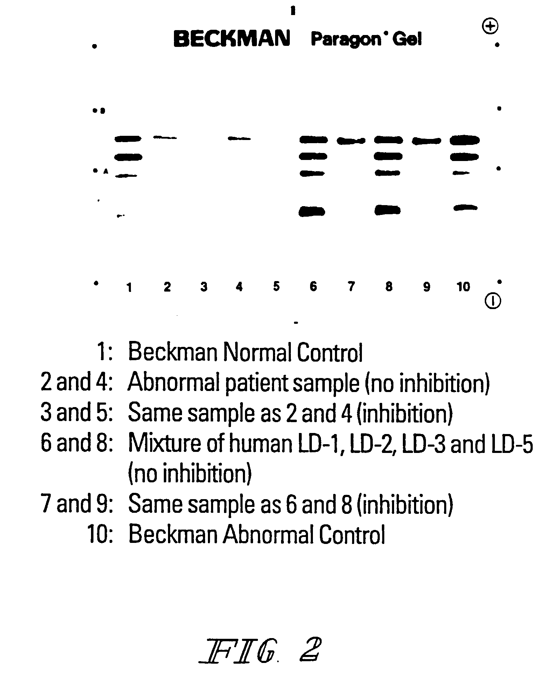

2. Experimental results of the Electrophoretic Analysis also indicate selectivity of the reagent of the present invention. FIG. 2 shows the Beckman Paragon Gel test results. Columns 2 and 4 are actual patient samples from patients with abnormal LDH profiles. Columns 3 and 5 show the same samples after inhibition with the reagent of the present invention. Columns 6 and 8 show profiles of mixtures of LD-1 through LD-3 and LD-5. Columns 7 and 9 show the same samples after treatment with the reagent of the present invention. FIGS. 3a and 3b show graphically the selectivity of the reagent of the present invention. FIG. 3a shows the profile of samples 2 and 4 of the Beckman Paragon Gel tests of FIG. 2. FIG. 3b shows the profile of samples 3 and 5, indicating that the reagent of the present invention selectively inhibited the activity of LD-2 through LD-5.

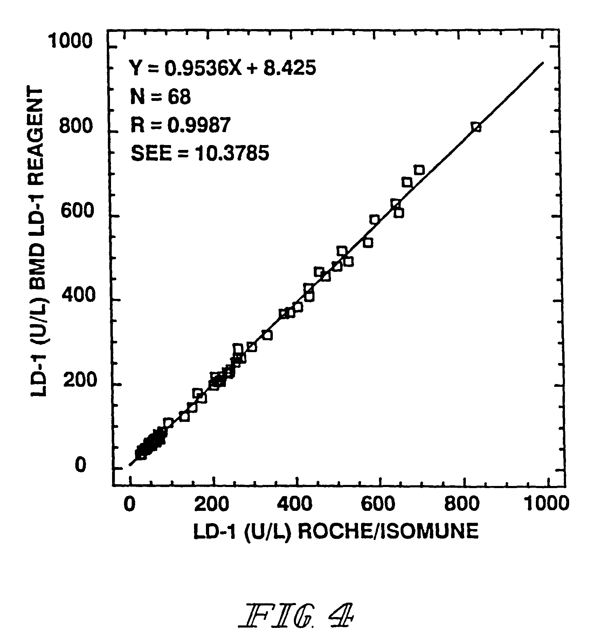

3. Accuracy of the reagent of the present invention was is further shown by a method comparison experiment. Sixty-eight patient serum samples from a local hospital were assayed by the Automated Analyzer method using the reagent of the present invention. The results are shown in the Y-axis of FIG. 4. The Roche Isomune LD separation system was used on separate alliquoits from the same samples. The results of this test are shown on the X-axis in FIG. 4. Linear regression analysis showed $Y=0.954x + 8.425$ ($R = 0.9987$).

4. Precision of the reagent of the present invention was indicated by a satirical analysis which is summarized below showing a low coefficient of variation (CV%) Samples of blood from normal patients and patients with heart problems (abnormal) were analyzed for LD-1 activity using the reagent of the present invention.

| Type Serum | No. of Tests | Mean (U/L) | Imprecision (CV%) | |
|------------|--------------|------------|------------|-------|
| | | | Within Run | Total |
| Normal | 60 | 141 | 1.2 | 2.6 |
| Abnormal | 60 | 571 | 0.5 | 0.8 |

Example 2

A. Materials

1. Reagent I: contains Lithium L-Lactate (90 mM), guanidine HCl (240 mM), sodium thiocyanate (240 mM) Tris buffer (119 mM) and $Na_2Co_3$ (12.5 mM) at pH 9.6.

2. Reagent II: contains Na(2)SO(4) (40 mM) and NAD (53.76 mM).
Reaction mixture is a combination of 2.0 ml of R-1, 0.5 ml of R-2 which has final reaction pH at 8.9.

3. LD isoenzymes: Same as described in Example 1.

B. Procedures using Automated Analyzer
Same as described in Example 1.

C. Manual Procedure
Same as described in Example 1.

D. Performance Characteristics
As shown in Table 2, the reagents in Example 2 are specific for the LD-1 isoenzyme of human lactate dehydrogenase, since full activities where recovered at various levels, with linearity up to at least 1000 U/L. In contrast, all other four isoenzymes have equal or less than 2.2% remaining activities which are not clinically significant. This result indicates that the reagent of the present invention can be the reaction product of a protein denaturing agent and thiocyanate anions.

Table 2

| SPECIFICITY FOR PURE INDIVIDUAL HUMAN LD ISOENZYMES AS SAMPLES USING A MIXTURE OF GUANIDINE HCL AND NaSCN | | |
|---|---|---|
| SAMPLES | TOTAL LDH REAGENT | LD-1 ISOENZYME REAGENT |
| LD-1 | U/L | U/L (Recovery) |
| 1X | 183 | 182 ( 99.5%) |
| 2X | 367 | 363 ( 98.9%) |
| 4X | 717 | 710 ( 99.0%) |
| 6X | 1060 | 1062 (102 %) |
| LD-2 | 781 | 16 ( 2.0%) |
| LD-3 | 1291 | 6 ( 0%) |
| LD-2,3,4,5* | 1357 | 0 (0%) |
| LD-5 | 1677 | 4 ( 0%) |

*This sample contains LD(2) LD(3) LD(4), and LD(5) isoenzymes with 43% of LD(4).

Comparative Example 3

A. Materials

1. Reagent I: contains Lithium L-Lactate (90 mM), guanidine-HCL Na(2)Co(3) (12.5mM), Tris buffer (119 mM) at pH 9.6.

2. Reagent II: contains Na(2)SO(4) (40 mM) and NAD (53.76 Mm).
Reaction mixture is a combination of 2.0 ml of R-1, 0.5 ml of R-2 which has final reaction reaction pH at 8.9.

3. LD isoenzymes: pure LD-1, LD-2, LD-3 and LD-5 individual isoenzymes are obtained from Aalto, Escondido, CA. Patient serum samples are obtained from a local hospital.

B. Procedures using Automated Analyzer
Same as described in Example 1.

C. Manual Procedure
Same as described in Example 1.

D. Performance Characteristics
This comparative example shows that a protein denaturing agent alone will not function as the reagent of the present invention.

Table

| SPECIFICITY FOR PURE INDIVIDUAL HUMAN LD ISOENZYMES AS SAMPLES USING A MIXTURE OF GUANIDINE HCL | | |
|---|---|---|
| SAMPLES | TOTAL LDH REAGENT | LD-1 ISOENZYME REAGENT |
| LD-1 | U/L | U/L (Recovery) |
| 1X | 183 | 202 (110 %) |
| 2X | 367 | 394 (107 %) |
| 4X | 717 | 759 (107 %) |
| 6X | 1060 | 1123 (106 %) |
| LD-2 | 781 | 826 (106 %) |
| LD-3 | 1291 | 1323 (102 %) |
| LD-2,3,4,5* | 1357 | 1102 ( 81.2 %) |
| LD-5 | 1677 | 159 ( 9.50%) |

*This sample contains LD(2), LD(3), LD(4), and LD(5) isoenzymes with 43% of LD(4).

Comparative Example 4

A. Materials

1. Reagent I: contains Lithium L-Lactate (90 mM), NaSCN (240 mM) Na(2)Co(3) (12.5 mM), Tris buffer (119 mM) at pH 9.6.

2. Reagent II: contains Na(2)SO(4) (40 mM) and NAD (53.76 Mm).
Reaction mixture is a combination of 2.0 ml of R-1, 0.5 ml of R-2 which has final reaction reaction pH at 8.9.

3. LD isoenzymes: pure LD-1, LD-2, LD-3 and LD-5 individual isoenzymes are obtained from Aalto, Escondido, CA. Patient serum samples are obtained from a local hospital.

B. Procedures using Automated Analyzer
Same as described in Example 1.

C. Manual Procedure
Same as described in Example 1.

D. Performance Characteristics
This comparative example shows that a protein denaturing agent alone will not function as the reagent of the present invention.

Table

| SPECIFICITY FOR PURE INDIVIDUAL HUMAN LD ISOENZYMES AS SAMPLES USING A NaSCN | | |
|---|---|---|
| SAMPLES | TOTAL LDH REAGENT | LD-1 ISOENZYME REAGENT |
| LD-1 | U/L | U/L (Recovery) |
| 1X | 183 | 252 (138 %) |
| 2X | 367 | 499 (136 %) |
| 4X | 717 | 964 (134 %) |
| 6X | 1060 | 1421 (134 %) |
| LD-2 | 781 | 905 (116 %) |
| LD-3 | 1291 | 1093 ( 85 %) |
| LD-2,3,4,5* | 1357 | 725 ( 53 %) |
| LD-5 | 1677 | 44 ( 2.6%) |

*This sample contains LD(2), LD(3), LD(4), and LD(5) isoenzymes with 43% of LD(4).

## Claims

1. A reagent for inactivating LD-2, LD-3, LD-4 and LD-5 in neutral to alkaline environments which comprises:

   (1) the reaction product of

       a) guanidine; and
       b) a thiocyanate anion, the reaction product being present in a concentration of about 240 mM and

   (2) lactate; and

   (3) a buffer to stabilize the pH at 9.5. to 9.6.

2. The reagent of claim 1 wherein the lactate is present at concentrations of from about 30 mM to about 120 mM.

3. A kit for determining LD-1 activity in a specimen containing LD-1, LD-2, LD-3, LD-4 and LD-5, the kit comprising the reagent of claim 1 for inactivating LD-2, LD-3, LD-4 and LD-5 in the specimen and a second reagent capable of reacting with the LD-1 which remains active in the specimen in a measurable manner.

4. The kit of claim 3 wherein the second reagent comprises lactate and NAD.

5. A method for determining LD-1 activity in a specimen containing LD-1, LD-2, LD-3, LD-4 and LD-5, which method comprises:

   applying the reagent of claim 1 to the specimen to inhibit the activity of LD-2, LD-3, LD-4 and LD-5;

   applying a second reagent to the specimen to react in a measurable manner with the remaining LD activity;

   determining the LD-1 activity in the specimen as a function of the measurable reaction of the second reagent.

6. The method of claim 5 wherein the second reagent comprises lactate and NAD.

7. A method for determining the LD-1 activity of a specimen as a ratio of total LD activity in the specimen, which method comprises:

   separating the specimen into first and second portions;

   applying the reagent of claim 1 to the first portion to inhibit the activity of LD-2, LD-3, LD-4 and LD-5;

   applying a second reagent to the first and second portions to react in a measurable manner to the LD activity in both the first and second portions; and

   determining the LD-1 activity in the specimen as a ratio of total LD activity in the specimen as a function of the measurable reaction of the second reagent with the first and second portions.

8. The method of claim 7 wherein the second reagent comprises lactate and NAD.

9. A kit for determining LD-1 activity as a ratio of total LD activity in a specimen, the kit comprising the reagent of claim 1 for inactivating LD-2, LD-3, LD-4 and LD-5 in one of two portions of the specimen and a second reagent capable of reacting in a measurable manner with LD-1, LD-2, LD-3, LD-4 and LD-5 in both portions of the specimen whereby the LD-1 activity as a ratio of total LD activity in the specimen can be determined as a function of the measurable reaction of the second reagent with both portions of the specimen.

10. The kit of claim 9 wherein the second reagent comprises lactate and NAD.

## EP 0 430 991 B1

**Patentansprüche**

1. Reagens zur Inaktivierung von LD-2, LD-3, LD-4 und LD-5 in neutraler bis alkalischer Umgebung, umfassend

   (1) das Reaktionsprodukt von

   (a) Guanidin, und
   (b) einem Thiocyanat-Anion,

   wobei das Reaktionsprodukt in einer Konzentration von etwa 240 mM vorhanden ist,
   (2) Lactat, und
   (3) einen Puffer zur Stabilisierung des pH auf 9,5 bis 9,6.

2. Reagens nach Anspruch 1, worin das Lactat in Konzentrationen von etwa 30 mM bis etwa 120 mM vorhanden ist.

3. Kit zur Bestimmung der LD-1-Aktivität in einer Probe, die LD-1, LD-2, LD-3, LD-4 und LD-5 enthält, wobei der Kit das Reagens nach Anspruch 1 zur Inaktivierung von LD-2, LD-3, LD-4 und LD-5 sowie ein zweites Reagens umfaßt, das mit dem LD-1, das in der Probe aktiv bleibt, in meßbarer Weise reagieren kann.

4. Kit nach Anspruch 3, wobei das zweite Reagens Lactat und NAD umfaßt.

5. Verfahren zur Bestimmung der LD-1-Aktivität in einer LD-1, LD-2, LD-3, LD-4 und LD-5 enthaltenden Probe, umfassend:

   Anwenden des Reagens nach Anspruch 1 auf die Probe, um die Aktivität von LD-2, LD-3, LD-4 und LD-5 zu hemmen;
   Anwenden eines zweiten Reagens auf die Probe, um in meßbarer Weise mit der verbleibenden LD-Aktivität zu reagieren;
   Bestimmen der LD-1-Aktivität in der Probe als Funktion der meßbaren Reaktion des zweiten Reagens.

6. Verfahren nach Anspruch 5, wobei das zweite Reagens Lactat und NAD umfaßt.

7. Verfahren zur Bestimmung der LD-1-Aktivität einer Probe als Verhältnis der Gesamt-LD-Aktivität in der Probe, umfassend:

   Trennen der Probe in einen ersten und zweiten Teil;
   Anwenden des Reagens nach Anspruch 1 auf den ersten Teil, um die Aktivität von LD-2, LD-3, LD-4 und LD-5 zu hemmen;
   Anwenden eines zweiten Reagens auf den ersten und zweiten Teil, um in meßbarer Weise auf die LD-Aktivität sowohl im ersten als auch im zweiten Teil zu reagieren; und
   Bestimmen der LD-I-Aktivität in der Probe als Verhältnis der Gesamt-LD-Aktivität in der Probe als Funktion der meßbaren Reaktion des zweiten Reagens mit dem ersten und zweiten Teil.

8. Verfahren nach Anspruch 7, wobei das zweite Reagens Lactat und NAD umfaßt.

9. Kit zur Bestimmung der LD-1-Aktivität als Verhältnis der Gesamt-LD-Aktivität in einer Probe, wobei der Kit das Reagens nach Anspruch 1 für die Inaktivierung von LD-2, LD-3, LD-4 und LD-5 in einem von zwei Teilen der Probe und ein zweites Reagens umfaßt, das in meßbarer Weise mit LD-1, LD-2, LD-3, LD-4 und LD-5 in beiden Teilen der Probe reagieren kann, wodurch die LD-I-Aktivität als Verhältnis der Gesamt-LD-Aktivität in der Probe als Funktion der meßbaren Reaktion des zweiten Reagens mit beiden Teilen der Probe bestimmt werden kann.

10. Kit nach Anspruch 9, wobei das zweite Reagens Lactat und NAD umfaßt.

**Revendications**

1. Réactif de désactivation de LD-2, LD-3, LD-4 et LD-5 dans des milieux neutres à basiques qui comprend:

(1) le produit réactionnel de:

(a) la guanidine; et
(b) d'un anion thiocyanate, le produit réactionnel étant présent à une concentration d'environ 240 mM et

(2) un lactate; et
(3) un tampon pour stabiliser le pH entre 9,5 et 9,6.

2. Réactif selon la revendication 1 dans lequel le lactate est présent à des concentrations comprises entre environ 30 mM et environ 120 mM.

3. Trousse de détermination de l'activité LD-1 dans un échantillon contenant LD-1, LD-2, LD-3, LD-4 et LD-5, la trousse comprenant le réactif de la revendication 1 pour la désactivation de LD-2, LD-3, LD-4 et LD-5 dans l'échantillon et un deuxième réactif capable de réagir avec LD-1 qui reste actif dans l'échantillon d'une manière mesurable.

4. Trousse de la revendication 3 dans laquelle le deuxième réactif comprend un lactate et NAD.

5. Procédé de détermination de l'activité de LD-1 dans un échantillon contenant LD-1, LD-2, LD-3, LD-4 et LD-5, lequel procédé consiste:

à appliquer le réactif selon la revendication 1 à l'échantillon afin d'inhiber l'activité de LD-2, LD-3, LD-4 et LD-5;
à appliquer un deuxième réactif à l'échantillon afin de le faire réagir d'une manière mesurable avec l'activité LD subsistante;
à déterminer l'activité LD-1 dans l'échantillon en fonction de la réaction mesurable du deuxième réactif.

6. Procédé selon la revendication 5, dans lequel le deuxième réactif comprend un lactate et NAD.

7. Procédé pour la détermination de l'activité de LD pour un échantillon en tant que rapport à l'activité totale LD dans l'échantillon, lequel procédé consiste:

à séparer l'échantillon en des première et deuxième portions;
à appliquer le réactif selon la revendication 1 à la première portion afin d'inhiber l'activité de LD-2, LD-3, LD-4 et LD-5;
à appliquer un deuxième réactif aux première et deuxième portions afin de le faire réagir d'une manière mesurable avec l'activité LD dans les première et deuxième portions à la fois; et
à déterminer l'activité LD-1 dans l'échantillon en tant que rapport à l'activité LD totale dans l'échantillon en fonction de la réaction mesurable du deuxième réactif avec les première et deuxième portions.

8. Procédé selon la revendication 7, dans lequel le deuxième réactif comprend un lactate et NAD.

9. Trousse de détermination de l'activité LD-1 en tant que rapport à l'activité LD totale dans un échantillon, la trousse comprenant le réactif selon la revendication 1 pour la désactivation de LD-2, LD-3, LD-4 et LD-5 dans une des deux portions de l'échantillon et un deuxième réactif capable de réagir d'une manière mesurable avec LD-1, LD-2, LD-3, LD-4 et LD-5 dans les deux portions de l'échantillon, grâce auquel on peut déterminer l'activité LD-1 en tant que rapport à l'activité LD totale dans l'échantillon en fonction de la réaction mesurable du deuxième réactif avec les deux portions de l'échantillon.

10. Trousse selon la revendication 9, dans laquelle le deuxième réactif comprend un lactate et NAD.

*FIG.1*

**BECKMAN** Paragon° Gel

1 2 3 4 5 6 7 8 9 10

1: Beckman Normal Control
2 and 4: Abnormal patient sample (no inhibition)
3 and 5: Same sample as 2 and 4 (inhibition)
6 and 8: Mixture of human LD-1, LD-2, LD-3 and LD-5 (no inhibition)
7 and 9: Same sample as 6 and 8 (inhibition)
10: Beckman Abnormal Control

# FIG. 2

*FIG. 3a*

| FRACTION | REL % |
|----------|-------|
| LD 1 | 52.8 |
| LD 2 | 21.2 |
| LD 3 | 8.0 |
| LD 4 | 4.2 |
| LD 5 | 13.8 |

*FIG. 3b*

| FRACTION | REL % |
|----------|-------|
| LD 1 | 98.3 |
| LD 2 | 0.6 |
| LD 3 | 0.1 |
| LD 4 | 0.8 |
| LD 5 | 0.2 |

*FIG. 4*